(19) **Europäisches Patentamt**
**European Patent Office**
**Office européen des brevets**

(11) **EP 4 574 806 A1**

(12) **EUROPÄISCHE PATENTANMELDUNG**

(43) Veröffentlichungstag:
    **25.06.2025 Patentblatt 2025/26**

(21) Anmeldenummer: **24218156.8**

(22) Anmeldetag: **06.12.2024**

(51) Internationale Patentklassifikation (IPC):
    **C07C 7/04** *(2006.01)*      **C07C 11/02** *(2006.01)*
    **C07C 45/50** *(2006.01)*     **C07C 47/02** *(2006.01)*
    **C07C 67/38** *(2006.01)*     **C07C 69/24** *(2006.01)*

(52) Gemeinsame Patentklassifikation (CPC):
    (C-Sets verfügbar)
    **C07C 7/04; C07C 45/50**                    (Forts.)

(84) Benannte Vertragsstaaten:
    **AL AT BE BG CH CY CZ DE DK EE ES FI FR GB GR HR HU IE IS IT LI LT LU LV MC ME MK MT NL NO PL PT RO RS SE SI SK SM TR**
    Benannte Erstreckungsstaaten:
    **BA**
    Benannte Validierungsstaaten:
    **GE KH MA MD TN**

(30) Priorität: **18.12.2023  EP 23217521**

(71) Anmelder: **Evonik Oxeno GmbH & Co. KG**
    **45772 Marl (DE)**

(72) Erfinder:
    • **Brächer, Alexander**
      **55546 Hackenheim (DE)**
    • **Franke, Robert**
      **45772 Marl (DE)**

    • **Sale, Anna Chiara**
      **40474 Düsseldorf (DE)**
    • **Fridag, Dirk**
      **45721 Haltern am See (DE)**
    • **Markovic, Ana**
      **45721 Haltern am See (DE)**
    • **Knossalla, Johannes**
      **48351 Everswinkel (DE)**
    • **Kucmierczyk, Peter**
      **44628 Herne (DE)**

(74) Vertreter: **Evonik Patent Association**
    **c/o Evonik Industries AG**
    **IP Management**
    **Postcode 84/339**
    **Rodenbacher Chaussee 4**
    **63457 Hanau (DE)**

(54) **VERFAHREN ZUR ZWEISTUFIGEN UMSETZUNG VON OLEFINEN MIT ZWISCHENGESCHALTETER DESTILLATION**

(57) Die vorliegende Erfindung betrifft ein Verfahren zur Umsetzung von Olefinen mit 4 bis 20 Kohlenstoffatomen, bei dem der eingesetzte Feedstrom, der zumindest lineare Olefine und verzweigte Olefine enthält, zwischen zwei Umsetzungsschritten destilliert wird und nur die dabei anfallende Schwersiederphase der zweiten Umsetzung, einer heterogen katalysierten Hydroformylierung, einer homogen katalysierten Hydroformylierung oder einer homogen katalysierten Alkoxycarbonylierung unterworfen wird.

EP 4 574 806 A1

(52) Gemeinsame Patentklassifikation (CPC): (Forts.)

C-Sets
**C07C 7/04, C07C 11/02;**
**C07C 45/50, C07C 47/02**

**Beschreibung**

Gegenstand der Erfindung ist ein Verfahren zur Umsetzung von Olefinen mit 4 bis 20 Kohlenstoffatomen, bei dem der eingesetzte Feedstrom, der zumindest lineare Olefine und verzweigte Olefine enthält, zwischen zwei Umsetzungsschritten destilliert wird und nur die dabei anfallende Schwersiederphase der zweiten Umsetzung, einer heterogen katalysierten Hydroformylierung, einer homogen katalysierten Hydroformylierung oder einer homogen katalysierten Alkoxycarbonylierung unterworfen wird.

Unter dem Begriff "Umsetzung" werden im Rahmen der vorliegenden Erfindung die drei unterschiedlichen Reaktionen heterogen katalysierte Hydroformylierung, homogen katalysierte Hydroformylierung und homogen katalysierte Alkoxycarbonylierung, bei denen die eingesetzten Feedströme, die die Olefine mit 4 bis 20 Kohlenstoffatome enthalten, umgesetzt werden, verstanden. Diese drei Reaktionstypen sind für sich bekannte Verfahren der organischen Chemie, werden im Rahmen der vorliegenden Erfindung jedoch ausführlicher beschrieben.

Bei der Hydroformylierung werden Olefine mit Synthesegas, d. h. einer Mischung aus Kohlenmonoxid (CO) und Wasserstoff ($H_2$), in Gegenwart eines geeigneten homogenen Katalysatorsystems zu den entsprechenden Aldehyden umgesetzt. Die Hydroformylierung ist ein großtechnisch genutztes Verfahren, welches in Anlagen betrieben wird, in denen die Aldehyde im Maßstab von mehreren bis hunderten Kilotonnen (kt) pro Jahr produziert werden können. Dabei typischerweise eingesetzte Katalysatorsysteme sind homogen gelöste Katalysatorsysteme und umfassen Übergangsmetallkomplexe aus zumeist Cobalt oder Rhodium als Metall und phosphorhaltigen Liganden. Beispiele dafür sind in der Patentliteratur zahlreich zu finden.

In den letzten Jahren wurden zudem wieder heterogen katalysierten Hydroformylierungen interessanter. Ein Beispiel für entsprechende Hydroformylierungen sind in der EP 3 632 885 A1 offenbart. Dort werden statt den bekannten homogen gelösten Katalysatoren Katalysatorsysteme eingesetzt, die heterogenisiert auf einem Monolith-Support aus einem porösen keramischen Material vorliegen. Die heterogenisierten Katalysatorsysteme sind insbesondere die aus der homogen katalysierten Hydroformylierung bekannten oder vergleichbare Übergangsmetallkomplexe aus zumeist Cobalt oder Rhodium als Metall und phosphorhaltigen Liganden.

Die Alkoxycarbonylierung ist die Umsetzung eines Olefins mit Kohlenmonoxid und einem Alkohol zu den daraus resultierenden Estern. Dabei werden üblicherweise Metall-Ligand-Komplexe als Katalysatoren eingesetzt, die homogen gelöst im Reaktionsgemisch und damit auch im Produktgemisch vorliegen. Ein entsprechendes Verfahren ist beispielsweise in der EP 3 750 620 A1 offenbart. Die eingesetzten Katalysatorsysteme umfassen insbesondere ein Metall aus der Gruppe 8 bis 10 des Periodensystems der Elemente (PSE), beispielsweise Palladium, oder eine Verbindung davon, einen phosphorhaltigen Liganden und eine Säure als Co-Katalysator.

Um die vorgenannten Umsetzungsverfahren, d. h. die homogen katalysierte Hydroformylierung, die heterogen katalysierte Hydroformylierung oder die homogen katalysierte Alkoxycarbonylierung, wirtschaftlich betreiben zu können kommt es auf verschiedene Faktoren an. Einer dieser Faktoren ist die Reaktionsgeschwindigkeit, die möglichst hoch sein sollte. Schließlich lässt sich im Vergleich zu einer Reaktion mit einer geringeren Reaktionsgeschwindigkeit pro Zeiteinheit mehr Produkt herstellen. Dabei ist aber zu beachten, dass sich bei einer Erhöhung der Reaktionsgeschwindigkeit andere Reaktionsparameter, wie der Reaktionsumsatz und/oder die Selektivität zum gewünschten Produkt sich nicht verschlechtern, damit sich der Vorteil der höheren Reaktionsgeschwindigkeit nicht ins Gegenteil verkehrt.

Ein Problem ergibt sich insbesondere bei zweistufigen Umsetzungsverfahren, wo in der ersten Stufe eine Umsetzung erfolgt und in der zweiten Stufe der Reststrom aus der ersten Stufe eingesetzt wird. Da in der ersten Stufe schon ein Teil der Olefine umgesetzt worden sind, kann in der zweiten Stufe die Reaktionsgeschwindigkeit absinken und/oder sich der Umsatz verringern.

Die Aufgabe der vorliegenden Erfindung war deshalb die Bereitstellung eines Verfahrens zur Umsetzung von Olefinen, mit dem in der zweiten Reaktionsstufe eine höhere Reaktionsgeschwindigkeit erzielt werden kann, während sich der Reaktionsumsatz und/oder die Selektivität zum gewünschten Produkt, hier ein Aldehyd bei der Hydroformylierung oder ein Ester bei der Alkoxycarbonylierung, nicht signifikant verschlechtern oder sogar verbessern.

Die Aufgabe konnte durch das Verfahren gemäß den Ansprüchen gelöst werden. Bevorzugte Ausgestaltungen sind in den abhängigen Ansprüchen angegeben. Das erfindungsgemäße Verfahren ist ein Verfahren zur Umsetzung von Olefinen mit 4 bis 20 Kohlenstoffatomen, welches die folgenden Schritte umfasst:

a) Bereitstellen eines Feedstroms, der zumindest lineare Olefine und verzweigte Olefine mit jeweils gleicher Anzahl an Kohlenstoffatomen umfasst;

b) Zuführen des Feedstroms aus Schritt a) zu einer Reaktionseinheit, die einen oder mehrere Reaktoren umfasst, und Durchführen einer Umsetzung in der Reaktionseinheit unter Erhalt eines Produktgemisches, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6-Alkohol ist,

c) Abtrennen eines Reststroms aus dem Produktgemisch aus Schritt b), wobei der Reststrom zumindest nicht umgesetzte Olefine umfasst, aus dem Reaktionsgemisch;

d) Destillation des Reststroms aus Schritt c) in mindestens einer Destillationskolonne unter Erhalt zumindest einer Leichtsiederphase und einer Schwersiederphase, wobei 1 Gew.-% bis weniger als 50 Gew.-% des destillierten Feedstroms als Leichtsiederphase anfällt und wobei der Anteil an linearen Olefinen in der Leichtsiederphase geringer ist als der Anteil der linearen Olefine in der Schwersiederphase; und

e) Zuführen der Schwersiederphase aus Schritt d) zu einer Reaktionseinheit, die einen oder mehrere Reaktoren umfasst, und Durchführen einer Umsetzung in der Reaktionseinheit, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6-Alkohol ist.

Der entscheidende Verfahrensschritt ist dabei Schritt d), die Destillation des Feedstroms und die ausschließliche Verwendung der Schwersiederphase für die Umsetzung in Schritt e). Das erfindungsgemäße Verfahren hat den Vorteil, dass die Reaktionsgeschwindigkeit in der Umsetzung in Schritt e) höher ist als bei den bekannten Verfahren, bei denen keine derartige Destillation erfolgt. Grund dafür dürfte sein, dass bei der Destillation eher unreaktivere Isomere und/oder reaktionshemmende Stoffe mit der Leichtsiederphase aus dem Feedstrom abgetrennt und damit nicht der Umsetzung zugeführt werden.

Der erste Schritt a) des erfindungsgemäßen Verfahrens ist die Bereitstellung eines Feedstroms umfassend die Olefine mit 4 bis 20 Kohlenstoffatomen, vorzugsweise Olefine mit 7 bis 16 Kohlenstoffatomen, besonders bevorzugt Olefine mit 7 bis 12 Kohlenstoffatomen. Ganz besonders bevorzugt sind Feedströme, die Olefine mit 8 und/oder 12 Kohlenstoffatomen umfassen. Die eingesetzten Feedströme sind üblicherweise technisch verfügbare Kohlenwasserstoffströme, die zumindest verschiedene Isomere des jeweiligen Olefins enthalten. Die Feedströme enthalten dabei zumindest lineare Olefine und verzweigte Olefine mit jeweils gleicher Anzahl an Kohlenstoffatomen. Die Menge an den jeweiligen Olefinen in den Kohlenwasserstoffströmen sollte verständlicherweise ausreichend hoch sein, um die Umsetzung in Schritt b) wirtschaftlich betreiben zu können, vorzugsweise sollten die Feedströme mindestens 5 Gew.-% der betreffenden Olefine enthalten, bezogen auf das Gesamtgewicht des Feedstroms.

Feedströme, die Olefine mit 4 Kohlenstoffatomen enthalten, beispielsweise Mischungen der verschiedenen Isomere des Butens (1-Buten, 2-Buten, Isobuten), sind üblicherweise Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische mit teils unterschiedlicher Konzentration an den Isomeren. Beispielsweise können für das erfindungsgemäße Verfahren geeignete Gemische linearer Butene aus der C4-Fraktion eines Steamcrackers gewonnen werden. Olefine mit 5 Kohlenstoffatomen, also Pentene, sind in Leichtbenzinfraktionen aus Raffinerien oder Crackern enthalten.

Die höheren Olefine können insbesondere durch Oligomerisierungsreaktionen, beispielsweise Dimerisierung, Trimerisierung oder Tetramerisierung, gewonnen werden. Geeignete

Kohlenwasserstoffströme sind weiterhin das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende isomere Hexadecen (Tetrabuten) sowie durch Cooligomerisierung von Olefinen mit unterschiedlicher Kohlenstoffatomanzahl (bevorzugt 2 bis 4 C-Atome) hergestellte Olefinmischungen, gegebenenfalls nach destillativer Trennung in Fraktionen mit gleicher oder unterschiedlicher C-Zahl. Weiterhin können Olefine oder Olefinmischungen, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden.

Die Feedströme, die in Schritt a) bereitgestellt werden, können vorher einem oder mehreren weiteren Verfahrensschritten unterworfen werden, um bestimmte Komponenten der Feedströme umzusetzen oder aus dem Strom zu entfernen. Ein Beispiel ist die Entfernung von Verunreinigungen, die schädlich für den Katalysator sein können, beispielsweise Sauerstoff-, Stickstoff- oder Schwefel-enthaltende Stoffe oder Verbindungen. Eine Möglichkeit zur Entfernung derartiger Verunreinigungen ist das Überleiten des Feedstroms über ein Adsorberbett, wobei die Verunreinigungen im Adsorber hängen bleiben. Solche Prozesse sind bekannt und zahlreich publiziert worden.

Der in Schritt a) bereitgestellte Feedstrom wird in Schritt b) einer Umsetzung in einer Reaktionseinheit zugeführt. Die Reaktionseinheit für die Umsetzung in Schritt b) kann aus einem oder mehreren Reaktoren bestehen. Der oder die Reaktor(en) können insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein. Sind mehrere Reaktoren vorhanden, können diese parallel oder in Reihe geschaltet oder einer Mischform aus paralleler und serieller Schaltung angeordnet sein.

Bei der Umsetzung in Schritt b) wird insbesondere kein Vollumsatz gefahren. In einer bevorzugten Ausführungsform der vorliegenden Erfindung wird die Umsetzung in Schritt b) so betrieben, dass der Umsatz bei der Umsetzung in Schritt b) maximal 95%, vorzugsweise maximal 92%, weiterhin bevorzugt maximal 90%, weiterhin bevorzugt maximal 88%, besonders bevorzugt maximal 85% beträgt. Je geringer die Umsätze sind, desto größer ist die Menge der nicht umgesetzten Olefine, die in der zweiten Stufe umgesetzt werden können. Auf der anderen Seite muss der Umsatz aber hoch genug sein, damit die erste Stufe wirtschaftlich betrieben werden kann.

Ist die Umsetzung in Schritt b) eine homogen katalysierte Reaktion sind folgende Verfahrensbedingungen bevorzugt:

Die bei dem Verfahren eingesetzten Olefine werden mit Synthesegas in Gegenwart eines homogen gelösten Katalysatorsystems hydroformyliert. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten Lösemittels durchgeführt werden.

Das bei der Hydroformylierung einsetzbare homogene Katalysatorsystem kann Co oder Rh, vorzugsweise Rh, und vorzugsweise einem phosphorhaltigen Liganden umfassen. Phosphorhaltige Liganden sind bei Co praktisch nicht unbedingt notwendig. Entsprechende Katalysatorsysteme sind dem Fachmann geläufig. In einer besonders bevorzugten Ausführungsform umfasst oder besteht das homogene Katalysatorsystem aus Rh und einem phosphorhaltigen Liganden. Geeignete Liganden für die erfindungsgemäßen Katalysatorsysteme sind dem Fachmann bekannt (siehe z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke).

Der phosphorhaltige Ligand für das erfindungsgemäße Katalysatorsystem ist vorzugsweise ein Phosphin (z. B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der homogen katalysierten Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 20 bis 350 bar, weiterhin bevorzugt im Bereich von 30 bis 325 bar und besonders bevorzugt im Bereich von 45 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und $H_2$, d. h. der Gesamtgasdruck ist dann der Synthesegasdruck.

Homogene katalysierte Hydroformylierungen können als Flüssigaustragsverfahren ("liquid recycle") oder als Gasaustragsverfahren ("gas recycle) betrieben werden. Beide Verfahrensvarianten sind dem Fachmann bekannt und in vielen Lehrbüchern beschrieben. Eine konkrete Auswahl eines solchen Verfahrens ist im Rahmen der vorliegenden Erfindung nicht notwendig, weil das Verfahren grundsätzlich auf beide Arten durchgeführt werden kann. Wichtig bei der homogenen Katalyse ist allenfalls noch die Abtrennung des Katalysatorsystems aus dem Reaktionsaustrag. Bei einem flüssigen Austrag ist dies beispielsweise über Flashverfahren oder Membrantrennung möglich. Bei einem gasförmigen Austrag beispielsweise mittels Kondensation und/oder Auswaschen. Auch dies ist dem Fachmann bekannt und darf keiner ausführlichen Erläuterung. Die weitere Aufarbeitung des Reaktionsaustrags, insbesondere die Abtrennung des Reaktionsprodukts, ist dem Fachmann ebenfalls geläufig und kann beispielsweise mittels eines thermischen Trennverfahrens wie der Destillation erfolgen. Thermische Trennung bzw. thermische Trennverfahren im Sinne der vorliegenden Erfindung meint ein Trennverfahren, bei dem die Trennung anhand des Siedepunktes erfolgt.

Ist die Umsetzung eine heterogen katalysierte Hydroformylierung sind folgende Verfahrensbedingungen bevorzugt:

Heterogen katalysierte Hydroformylierungen sind im Rahmen der vorliegenden Erfindung insbesondere solche, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass das Katalysatorsystem durch Ausbildung eines dünnen Flüssigkeitsfilms auf der Oberfläche und/oder in den Poren eines festen Trägermaterials immobilisiert vorliegt.

Die heterogen katalysierte Hydroformylierung zeichnet sich insbesondere dadurch aus, dass die Schwersiederphase aus Schritt d) gasförmig über einen Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, einen Stabilisator umfasst, heterogenisiert vorliegt, geleitet wird.

Die Temperatur bei der heterogen katalysierten Hydroformylierung kann im Bereich von 65 bis 200 °C, vorzugsweise 75 bis 175 °C und besonders bevorzugt 85 bis 150 °C liegen. Der Druck bei der heterogen katalysierten Hydroformylierung sollte größer als 0 sein, aber insbesondere nicht größer als 35 bar, vorzugsweise nicht größer als 30 bar, besonders bevorzugt nicht größer 25 bar sein. Das molare Verhältnis zwischen Synthesegas und dem Einsatzgemisch sollte zwischen 6:1 und 1:1, vorzugsweise zwischen 5:1 und 3:1 liegen. Optional kann das Einsatzgemisch mit Inertgas oder einem Lösemittel verdünnt werden, beispielsweise mit den in technischen Kohlenwasserstoffströmen befindlichen Alkanen, um die Reaktion zu kontrollieren.

Das bei dem erfindungsgemäßen Hydroformylierungsverfahren eingesetzte Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, besonders bevorzugt Cobalt und Rhodium, mindestens einen organischen Phosphor-enthaltenden Liganden, einen Stabilisator und optional eine ionische Flüssigkeit.

Der Stabilisator ist vorzugsweise eine organische Aminverbindung, besonders bevorzugt eine organische Aminverbindung, die mindestens eine 2,2,6,6-Tetramethylpiperidineinheit enthält.

Der organische Phosphor-enthaltende Ligand für das erfindungsgemäße Katalysatorsystem weist vorzugsweise die allgemeine Formel (I)

$$R'\text{-}A\text{-}R''\text{-}A\text{-}R''' \qquad (I)$$

wobei R', R" und R''' jeweils organische Reste sind und beide A jeweils eine brückende -O-P(-O)2-Gruppe sind, wobei zwei der drei Sauerstoffatome -O- jeweils an Rest R' und den Rest R''' gebunden sind, mit der Maßgabe das R' und R''' zwei voneinander getrennte organische Reste sind. R' und R''' können den gleichen oder einen unterschiedlichen organischen Rest darstellen. Die organischen Reste R', R" und R''' enthalten vorzugsweise keine endständige Trialkoxysilangruppe.

In einer bevorzugten Ausführungsform sind R', R" und R''' in der Verbindung der Formel (I), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R''' nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe. Ein Beispiel für einen geeigneten Liganden ist Biphephos (6,6'-[(3,3'-Di-tert-butyl-5,5'-dimethoxy-1,1'-biphenyl-2,2'-diyl)bis(oxy)]bis(dibenzo[d,f][1,3,2]dioxaphosphepin)).

Das poröse keramische Material, aus dem der Support besteht, ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon. Die Silikatkeramik ist vorzugsweise ausgewählt aus Alumosilikat, Magnesiumsilikat, und Mischungen davon, wie z. B. Betonit. Die oxidische Keramik ist vorzugsweise ausgewählt aus $\gamma$-Aluminiumoxid, $\alpha$-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik.

Der Support kann ein Monolith sein, d. h. der Support kann aus einem Block (ein dreidimensionales Objekt) aus einem keramischen Material bestehen. Ein solcher Block kann sowohl einstückig ausgebildet sein als auch aus mehreren, also mindestens zwei Einzelteilen bestehen, die zu dem Block zusammengefügt werden können und/oder miteinander fest oder lösbar verbunden sind. Der Support kann aber auch als in Form eines Granulats oder in Form von Pellets vorliegen. Der mittlere Partikeldurchmesser (d50) des Supports kann dann von 0,1 mm bis 7 mm, vorzugsweise 0,3 bis 6 mm, besonders bevorzugt von 0,5 mm bis 5 mm betragen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren ermittelt werden. Die Herstellung des Supports, in Form eines Granulats oder in Form von Pellets kann nach dem Fachmann bekannten Verfahren erfolgen. Beispielsweise könnte es dadurch erfolgen, dass ein Monolith aus dem carbidischen, nitridischen, silicidischen Material oder Mischungen davon mechanisch zerkleinert wird, beispielsweise mit einem Backenbrecher, und die Partikelgröße des erhaltenen Bruchgranulats mittels Sieben eingestellt wird.

Der Support besteht erfindungsgemäß aus einem porösen keramischen Material, das keramische Material weist also Poren auf. Grundsätzlich sind verschiedene Porositäten oder Porendurchmesser denkbar. Der Porendurchmesser liegt jedoch vorzugsweise im Bereich von 0,9 nm bis 30 $\mu$m, weiterhin bevorzugt im Bereich von 10 nm bis 25 $\mu$m und besonders bevorzugt im Bereich von 70 nm bis 20 $\mu$m. Der Porendurchmesser kann mittels Stickstoffadsorption oder Quecksilber-Porosimetrie gemäß DIN 66133 (Stand: 1993-06) bestimmt werden.

Auf den Support aus dem keramischen Material kann zusätzlich ein sogenannter Washcoat aufgetragen werden, welcher bezogen auf das keramische Material des Supports aus dem gleichen oder einem unterschiedlichen Keramikmaterial besteht, insbesondere ein aus den vorgenannten Keramikmaterialen ausgewähltes Keramikmaterial, vorzugsweise Siliciumoxid. Der Washcoat selber kann porös oder unporös sein, vorzugsweise ist der Waschcoat unporös. Die Partikelgröße des Washcoats beträgt vorzugsweise 5 nm bis 3 μm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports. Auf den so erzeugten keramischen Support mit dem aufgebrachten Washcoat wird dann das Katalysatorsystem aufgebracht. Es ist jedoch bevorzugt, dass der Support keinen Washcoat umfasst.

Der bei der heterogen katalysierten Hydroformylierung erhaltene Reaktionsaustrag muss grundsätzlich nicht vom Katalysatorsystem befreit werden. Die weitere Aufarbeitung, also beispielsweise die Abtrennung der Reaktionsprodukte, ist dem Fachmann geläufig und kann grundsätzlich destillativ oder nach einem anderen thermischen Trennverfahren erfolgen.

Ist die Umsetzung eine homogen katalysierte Alkoxycarbonylierung sind folgende Verfahrensbedingungen bevorzugt:

Bei der Alkoxycarbonylierung werden Olefine zusammen mit Kohlenmonoxid (CO) und einem Alkohol, im vorliegenden Fall einem C1- bis C6-Alkohol, in Anwesenheit eines homogen katalysierten Katalysatorsystems zur Reaktion gebracht und es entstehen Ester.

Das Kohlenmonoxid kann dabei direkt als Einsatzgemisch oder durch die Zugabe eines Kohlenmonoxidhaltigen Gases, ausgewählt aus Synthesegas, Wassergas, Generatorgas und anderen Kohlenmonoxidhaltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei Alkoxycarbonylierung eingesetzte Alkohol ist ein Mono- oder Polyalkohol (Polyalkohol = zwei oder mehr OH-Gruppen) mit 1 bis 6 Kohlenstoffatomen. Geeignete Alkohole für die Umsetzung in Schritt e) sind Methanol, Ethanol, 1-Propanol, 1-Butanol, 1-Pentanol, 1-Hexanol, 2-Propanol, tert-Butanol, 3-Pentanol, Cyclohexanol, Phenol oder Mischungen daraus, Bevorzugt werden Methanol und Ethanol bei der Alkoxycarbonylierung eingesetzt. Wird Methanol eingesetzt, wird die Umsetzung auch als Methoxycarbonylierung bezeichnet. Bei Einsatz von Ethanol wird die Umsetzung auch als Ethoxycarbonylierung bezeichnet.

Das für die Alkoxycarbonylierung eingesetzte homogene Katalysatorsystem umfasst vorzugsweise zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladium-chlorid [$PdCl_2$], Palladium(II)-Acetylacetonat [$Pd(acac)_2$], Palladium(II)-Acetat [$Pd(OAc)_2$], Dichloro-(1,5-cyclooctadien)palladium(II) [$Pd(cod)_2Cl_2$], Bis(dibenzylideneaceton)palladium(0) [$Pd(dba)_2$], Tris(dibenzylideneaceton)dipalladium(0) [$Pd_2(dba)_3$] Bis(acetonitril)-dichloropalladium(II) [$Pd(CH_3CN)_2Cl_2$], Palladium(cinnamyl)-dichlorid [$Pd(cinnamyl)Cl_2$]. Vorzugsweise kommen die Verbindungen [$Pd(acac)_2$] oder [$Pd(OAc)_2$] zum Einsatz. Die Metallkonzentration von Palladium bei der Alkoxycarbonylierung beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann bei der Alkoxycarbonylierung 1 : 1 bis 10 : 1, vorzugsweise 2 : 1 bis 6 : 1, besonders bevorzugt 3 : 1 bis 5 : 1 betragen.

Das homogene Katalysatorsystem umfasst bei der Alkoxycarbonylierung weiterhin eine Säure, wobei es sich insbesondere um eine Brönsted- oder eine Lewis-Säure handeln kann. Als Lewis-Säure können insbesondere Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure. Carbonsäure sind hingegen weniger bis gar nicht geeignet.

Die homogen katalysierte Alkoxycarbonylierung wird vorzugsweise bei einer Temperatur von 25 bis 140 °C, weiterhin bevorzugt bei einer Temperatur von 60 bis 120 °C und besonders bevorzugt bei einer Temperatur von 70 bis 110 °C durchgeführt. Der Druck kann zwischen 5 und 60 bar, vorzugsweise zwischen 10 und 40 bar, besonders bevorzugt zwischen 15 und 30 bar betragen.

Durch die homogen katalysierte Alkoxycarbonylierung wird ein Produktgemisch erhalten, das zumindest den durch die Umsetzung gebildeten Ester, das homogene Katalysatorsystem, Leichtsieder, beispielsweise leichtsiedende Nebenprodukte wie Ether, Hochsieder, nicht umgesetzte Alkohole und ggf. nicht umgesetzte Kohlenwasserstoffe umfasst. Das Produktgemisch kann deshalb einer nachfolgenden Katalysatorabtrennung unterworfen werden. Dies kann beispielsweise mit einer Membrantrennung erfolgen, wodurch das homogene Katalysatorsystem und nicht umgesetzter Kohlenwasserstoff und/oder nicht umgesetzter Alkohol im Retentat angereichert, während der gebildete Ester im Permeat angereichert wird. Das Retentat, welches das angereicherte homogene Katalysatorsystem umfasst, kann in die Reaktionszone zurückgeführt werden.

Die weitere Aufarbeitung des Permeats, insbesondere die Abtrennung der Ester als Zielprodukte, kann nach bekannten Verfahren erfolgen und ist dem Fachmann grundsätzlich geläufig. Eine Möglichkeit sind thermische Trennverfahren wie die Destillation.

Bei der Umsetzung in Schritt b) entsteht ein Produktgemisch, das aus der Reaktionseinheit entnommen und zur Abtrennung in Schritt c) geführt wird. Das Produktgemisch wird zumindest das gebildete Reaktionsprodukt und nicht umgesetzte Olefine umfassen. Bei der Abtrennung in Schritt c) wird ein Reststrom, der zumindest nicht umgesetzte Olefine umfasst, aus dem Produktgemisch abgetrennt. Durch die Abtrennung in Schritt c) wird demzufolge zumindest ein Teil der gebildeten Reaktionsprodukte von den nicht umgesetzten Olefinen abgetrennt. Die Abtrennung in Schritt c) erfolgt vorzugsweise mittels thermischer Trennung.

Die thermische Trennung bei der Abtrennung in Schritt c) kann eine Destillation, eine Dünnschichtverdampfung, eine Fallfilmverdampfung oder eine Kombination von zwei oder mehr davon sein. Der Fachmann kann aufgrund seines Fachwissens aber entsprechend den Anforderungen des jeweiligen Verfahrens ein geeignetes Verfahren wählen. In einer Ausführungsform der vorliegenden Erfindung ist die thermische Trennung eine Dünnschichtverdampfung, eine Fallfilmverdampfung oder eine Kombination von Dünnschichtverdampfung und Fallfilmverdampfung. Dünnschichtverdampfung und Fallfilmverdampfung können auch mehrstufig ausgestaltet sein, also als eine sogenannte Kaskade vorliegen. In einer besonders bevorzugten Ausführungsform ist die thermische Trennung eine mehrstufige Fallfilmverdampfung (Verdampferkaskade). Wird zur thermischen Trennung eine Dünnschichtverdampfung, eine Fallfilmverdampfung oder eine Kombination daraus eingesetzt muss nach dem ersten Membrantrennschritt ins Vakuum, also einen Druck von $\geq$ 10 mbar aber weniger als 1 bar, entspannt werden. Eine solche Entspannung erfolgt vorzugsweise in einem Schritt.

Durch die Abtrennung des Produktgemisches in Schritt c) fällt ein Reststrom an, der zumindest nicht umgesetzte Olefine enthält. Es fällt zudem ein Produktstrom an, der zumindest die gebildeten Reaktionsprodukte enthält, an. Dieser Produktstrom ist für die vorliegende Erfindung nicht weiter relevant. Der Produktstrom kann zu einer weiteren Aufarbeitung und oder einer weiteren Umsetzung geführt werden, beispielsweise zu einer Hydrierung, wo die durch eine Hydroformylierung entstandene Aldehyde zu den entsprechenden Alkoholen umgesetzt werden.

Der in Schritt c) abgetrennte Reststrom wird im nachfolgenden Schritt d) einer Destillation unterworfen, bei der eine Schwersiederphase und eine Leichtsiederphase anfallen. Die Destillation in Schritt d) wird vorzugsweise bei einer Kopftemperatur im Bereich von 40 bis 100 °C, besonders bevorzugt im Bereich von 45 bis 80 °C durchgeführt. Der Druck am Kopf beträgt bei der Destillation in Schritt d) vorzugsweise 50 bis 400 mbar, besonders bevorzugt 80 bis 350 mbar. Die Temperatur im Sumpf beträgt bei der Destillation in Schritt d) vorzugsweise 60 bis 150 °C, besonders bevorzugt 70 bis 140 °C. Der Druck im Sumpf beträgt bei der Destillation in Schritt d) vorzugsweise 70 bis 500 mbar, besonders bevorzugt 90 bis 450 mbar. Die für eine bestimmte Trennaufgabe notwendigen bzw. vorliegenden Temperaturen sind bei der Destillation bekanntermaßen vom Druck abhängig. Der Fachmann wird anhand der durchzuführenden Trennaufgabe eine geeignete Parameterkombination aus Druck und Temperatur wählen können.

Bei der Destillation können unreaktivere Isomere und/oder reaktionshemmende Stoffe zumindest teilweise in die Leichtsiederphase übergehen, wodurch die Umsetzung der Schwersiederphase in Schritt e) mit höherer Rektionsgeschwindigkeit erfolgen kann. Bei der Destillation in Schritt d) fallen 1 Gew.-% bis weniger als 50 Gew.-%, vorzugsweise 5 Gew.-% bis 40 Gew.-%, besonders bevorzugt 10 Gew.-% bis 30 Gew.-% des destillierten Feedstroms als Leichtsiederphase an. Die Abtrennung der Leichtsiederphase in der Destillation kann dabei auf unterschiedliche Art erfolgen, was dem Fachmann grundsätzlich geläufig ist. So kann die Abtrennung der bestimmten Menge der Leichtsiederphase beispielsweise über die Trennschärfe der Destillation, die anhand des Rücklaufverhältnisses und/oder der Temperatur beeinflusst werden kann, erfolgen. Möglich wäre auch eine Abtrennung nach der abgetrennten Masse, also beispielsweise einem Massenmesser oder ähnlichen geeigneten Apparaturen oder Einbauten. Der Anteil oder die Menge an Feedstrom, der oder die als Leichtsiederphase in der Destillation in Schritt d) abgetrennt wird, kann aber auch anhand anderer Parameter eingestellt werden.

Die Folge der Destillation in Schritt d) ist, dass der Anteil an linearen Isomeren der eingesetzten Olefine in der Leichtsiederphase geringer ist als der Anteil der linearen Isomeren der eingesetzten Olefine in der Schwersiederphase. Dies kann beispielsweise mittels NMR oder Gaschromatographie überprüft bzw. ermittelt werden. Die verzweigten Isomere sind üblicherweise leichtsiedender und gehen in größerem Anteil in die Leichtsiederphase über. Da es sich im Rahmen der vorliegenden Erfindung bei den eingesetzten Olefinen in der Regel um ein Gemisch isomerer Olefine handelt, ist es kaum zu verhindern, dass auch lineare Isomere in die Leichtsiederphase übergehen. Die Destillation sollte jedoch nur in geeigneten Bedingungen durchgeführt werden, um zu ermöglichen, dass der Anteil der linearen Isomere in der Schwersiederphase höher ist.

Für die erfindungsgemäße Destillation in Schritt d) eignen sich grundsätzlich alle bekannten Destillationskolonnen. Diese Destillationskolonnen weisen typischerweise Einbauten zur Verbesserung der Trennschärfe auf. Geeignete Einbauten sind zum Beispiel Böden, unstrukturierte Packungen (Füllkörper) oder strukturierte Packungen. Als Böden werden üblicherweise Glockenböden, Siebböden, Ventilböden mit festen oder beweglichen Ventilen, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel, SuperRinge/SuperRinge Plus oder Intalox®-Sättel verwendet. Strukturierte Packungen werden zum Beispiel unter dem Handelsnamen Mellapak® der Firma Sulzer vertrieben. Neben den genannten Einbauten sind dem Fachmann weitere geeignete Einbauten bekannt und können ebenfalls verwendet werden. In einer bevorzugten Ausführungsform umfasst die mindestens eine Destillationskolonne in Schritt c) 10 bis 100 Böden, bevorzugt 15 bis 80 Böden.

Die Bedingungen der Destillation sind abhängig von der Feedzusammensetzung und in weiten Bereichen variabel. Für sehr reine Ströme, die nur geringe Anteile an oder keine Verbindungen mit mehr oder weniger Kohlenstoffatomen aufweisen, reicht üblicherweise eine einstufige Destillation aus. Im Sumpf fallen bei der Destillation dann die eher linearen Isomere an, die im Schritt e) umgesetzt werden sollen. Am Kopf der einstufigen Destillation fallen die eher verzweigten Isomere an, die abgetrennt werden. Enthalten die eingesetzten Feedströme signifikante Mengen an Verbindungen mit langkettigen Kohlenstoffatomen, kann eine mehrstufige Destillation von Vorteil sein, bei der der gewünschte Strom für den Schritt e) als Kopfstrom der zweiten bzw. letzten Destillationskolonne abgenommen wird.

Die aus der Destillation in Schritt d) erhaltene Phase wird dann in Schritt e) einer Umsetzung in einer Reaktionseinheit zugeführt. Die Reaktionseinheit für die Umsetzung in Schritt e) kann aus einem oder mehreren Reaktoren bestehen. Der oder die Reaktor(en) können insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein. Sind mehrere Reaktoren vorhanden, können diese parallel oder in Reihe geschaltet oder einer Mischform aus paralleler und serieller Schaltung angeordnet sein.

Ist die Umsetzung eine homogen katalysierte Reaktion sind folgende Verfahrensbedingungen bevorzugt: Die bei dem Verfahren eingesetzten Olefine werden mit Synthesegas in Gegenwart eines homogen gelösten Katalysatorsystems hydroformyliert. Das molare Verhältnis zwischen Synthesegas und dem Einsatzgemisch sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten Lösemittels durchgeführt werden.

Das bei der Hydroformylierung einsetzbare homogene Katalysatorsystem kann Co oder Rh, vorzugsweise Rh, und vorzugsweise einem phosphorhaltigen Liganden umfassen. Phosphorhaltige Liganden sind bei Co praktisch nicht unbedingt notwendig. Entsprechende Katalysatorsysteme sind dem Fachmann geläufig. In einer besonders bevorzugten Ausführungsform umfasst oder besteht das homogene Katalysatorsystem aus Rh und einem phosphorhaltigen Liganden. Geeignete Liganden für die erfindungsgemäßen Katalysatorsysteme sind dem Fachmann bekannt (siehe z. B. die Lehrbücher "Rhodium Catalyzed Hydroformylation" (aus 2002) von P. W. N. M van Leeuwen oder "Hydrofomylation - Fundamentals, Proceses and Applications in Organic Synthesis" (aus 2016) von A. Börner und R. Franke).

Der phosphorhaltige Ligand für das erfindungsgemäße Katalysatorsystem ist vorzugsweise ein Phosphin (z. B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der homogen katalysierten Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 20 bis 350 bar, weiterhin bevorzugt im Bereich von 30 bis 325 bar und besonders bevorzugt im Bereich von 45 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und $H_2$, d. h. der Gesamtgasdruck ist dann der Synthesegasdruck.

Homogene katalysierte Hydroformylierungen können als Flüssigaustragsverfahren ("liquid recycle") oder als Gasaustragsverfahren ("gas recycle) betrieben werden. Beide Verfahrensvarianten sind dem Fachmann bekannt und in vielen Lehrbüchern beschrieben. Eine konkrete Auswahl eines solchen Verfahrens ist im Rahmen der vorliegenden Erfindung nicht notwendig, weil das Verfahren grundsätzlich auf beide Arten durchgeführt werden kann. Wichtig bei der homogenen Katalyse ist allenfalls noch die Abtrennung des Katalysatorsystems aus dem Reaktionsaustrag. Bei einem flüssigen Austrag ist dies beispielsweise über Flashverfahren oder Membrantrennung möglich. Bei einem gasförmigen Austrag beispielsweise mittels Kondensation und/oder Auswaschen. Auch dies ist dem Fachmann bekannt und darf keiner ausführlichen Erläuterung. Die weitere Aufarbeitung des Reaktionsaustrags, insbesondere die Abtrennung des Reaktionsprodukts, ist dem Fachmann ebenfalls geläufig und kann beispielsweise mittels eines thermischen Trennverfahrens wie der Destillation erfolgen. Thermische Trennung bzw. thermische Trennverfahren im Sinne der vorliegenden Erfindung meint ein Trennverfahren, bei dem die Trennung anhand des Siedepunktes erfolgt.

Ist die Umsetzung eine heterogen katalysierte Hydroformylierung sind folgende Verfahrensbedingungen bevorzugt:

Heterogen katalysierte Hydroformylierungen sind im Rahmen der vorliegenden Erfindung insbesondere solche, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass das Katalysatorsystem durch Ausbildung eines dünnen Flüssigkeitsfilms auf der Oberfläche und/oder in den Poren eines festen Trägermaterials immobilisiert vorliegt.

Die heterogen katalysierte Hydroformylierung zeichnet sich insbesondere dadurch aus, dass die Schwersiederphase aus Schritt d) gasförmig über einen Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, einen Stabilisator umfasst, heterogenisiert vorliegt, geleitet wird.

Die Temperatur bei der heterogen katalysierten Hydroformylierung kann im Bereich von 65 bis 200 °C, vorzugsweise 75 bis 175 °C und besonders bevorzugt 85 bis 150 °C liegen. Der Druck bei der heterogen katalysierten Hydroformylierung sollte größer als 0 sein, aber insbesondere nicht größer als 35 bar, vorzugsweise nicht größer als 30 bar, besonders bevorzugt nicht größer 25 bar sein. Das molare Verhältnis zwischen Synthesegas und dem Einsatzgemisch sollte zwischen 6:1 und 1:1, vorzugsweise zwischen 5:1 und 3:1 liegen. Optional kann das Einsatzgemisch mit Inertgas oder einem Lösemittel verdünnt werden, beispielsweise mit den in technischen Kohlenwasserstoffströmen befindlichen Alkanen, um die Reaktion zu kontrollieren.

Das bei dem erfindungsgemäßen Hydroformylierungsverfahren eingesetzte Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, besonders bevorzugt Cobalt und Rhodium, mindestens einen organischen Phosphor-enthaltenden Liganden, einen Stabilisator und optional eine ionische Flüssigkeit.

Der Stabilisator ist vorzugsweise eine organische Aminverbindung, besonders bevorzugt eine organische Aminverbindung, die mindestens eine 2,2,6,6-Tetramethylpiperidineinheit enthält.

Der organische Phosphor-enthaltende Ligand für das erfindungsgemäße Katalysatorsystem weist vorzugsweise die allgemeine Formel (I)

$$R'\text{-}A\text{-} R''\text{-}A\text{-} R''' \qquad\qquad (I)$$

wobei R', R" und R''' jeweils organische Reste sind und beide A jeweils eine brückende -O-P(-O)2-Gruppe sind, wobei zwei der drei Sauerstoffatome -O- jeweils an Rest R' und den Rest R''' gebunden sind, mit der Maßgabe das R' und R''' nicht identisch sind. Die organischen Reste R', R" und R''' enthalten vorzugsweise keine endständige Trialkoxysilangruppe.

In einer bevorzugten Ausführungsform sind R', R" und R''' in der Verbindung der Formel (I), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R''' nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Das poröse keramische Material, aus dem der Support besteht, ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon. Die Silikatkeramik ist vorzugsweise ausgewählt aus Alumosilikat, Magnesiumsilikat, und Mischungen davon, wie z. B. Betonit. Die oxidische Keramik ist vorzugsweise ausgewählt aus γ-Aluminiumoxid, α-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Siliciumnitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik.

Der Support kann ein Monolith sein, d. h. der Support kann aus einem Block (ein dreidimensionales Objekt) aus einem keramischen Material bestehen. Ein solcher Block kann sowohl einstückig ausgebildet sein als auch aus mehreren, also mindestens zwei Einzelteilen bestehen, die zu dem Block zusammengefügt werden können und/oder miteinander fest oder lösbar verbunden sind. Der Support kann aber auch als in Form eines Granulats oder in Form von Pellets vorliegen. Der mittlere Partikeldurchmesser (d50) des Supports kann dann von 0,1 mm bis 7 mm, vorzugsweise 0,3 bis 6 mm, besonders bevorzugt von 0,5 mm bis 5 mm betragen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren ermittelt werden. Die Herstellung des Supports, in Form eines Granulats oder in Form von Pellets kann nach dem Fachmann bekannten Verfahren erfolgen. Beispielsweise könnte es dadurch erfolgen, dass ein Monolith aus dem carbidischen, nitridischen, silicidischen Material oder Mischungen davon mechanisch zerkleinert wird, beispielsweise mit einem Backenbrecher, und die Partikelgröße des erhaltenen Bruchgranulats mittels Sieben eingestellt wird.

Der Support besteht erfindungsgemäß aus einem porösen keramischen Material, das keramische Material weist also Poren auf. Grundsätzlich sind verschiedene Porositäten oder Porendurchmesser denkbar. Der Porendurchmesser liegt jedoch vorzugsweise im Bereich von 0,9 nm bis 30 μm, weiterhin bevorzugt im Bereich von 10 nm bis 25 μm und besonders bevorzugt im Bereich von 70 nm bis 20 μm. Der Porendurchmesser kann mittels Stickstoffadsorption oder Quecksilber-Porosimetrie gemäß DIN 66133 (Stand: 1993-06) bestimmt werden.

Auf den Support aus dem keramischen Material kann zusätzlich ein sogenannter Washcoat aufgetragen werden, welcher bezogen auf das keramische Material des Supports aus dem gleichen oder einem unterschiedlichen Keramikmaterial besteht, insbesondere ein aus den vorgenannten Keramikmaterialen ausgewähltes Keramikmaterial, vorzugsweise Siliciumoxid. Der Washcoat selber kann porös oder unporös sein, vorzugsweise ist der Waschcoat unporös. Die Partikelgröße des Washcoats beträgt vorzugsweise 5 nm bis 3 μm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports. Auf den so erzeugten keramischen Support mit dem aufgebrachten Washcoat wird dann das Katalysatorsystem aufgebracht. Es ist jedoch bevorzugt, dass der Support keinen Washcoat umfasst.

Der bei der heterogen katalysierten Hydroformylierung erhaltene Reaktionsaustrag muss grundsätzlich nicht vom Katalysatorsystem befreit werden. Die weitere Aufarbeitung, also beispielsweise die Abtrennung der Reaktionsprodukte, ist dem Fachmann geläufig und kann grundsätzlich destillativ oder nach einem anderen thermischen Trennverfahren erfolgen.

Ist die Umsetzung eine homogen katalysierte Alkoxycarbonylierung sind folgende Verfahrensbedingungen bevorzugt:

Bei der Alkoxycarbonylierung werden Olefine zusammen mit Kohlenmonoxid (CO) und einem Alkohol, im vorliegenden Fall einem C1- bis C6-Alkohol, in Anwesenheit eines homogen katalysierten Katalysatorsystems zur Reaktion gebracht und es entstehen Ester.

Das Kohlenmonoxid kann dabei direkt als Einsatzgemisch oder durch die Zugabe eines Kohlenmonoxidhaltigen Gases, ausgewählt aus Synthesegas, Wassergas, Generatorgas und anderen Kohlenmonoxidhaltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei Alkoxycarbonylierung eingesetzte Alkohol ist ein Mono- oder Polyalkohol (Polyalkohol = zwei oder mehr OH-Gruppen) mit 1 bis 6 Kohlenstoffatomen. Geeignete Alkohole für die Umsetzung in Schritt b) oder e) sind Methanol, Ethanol, 1-Propanol, 1-Butanol, 1-Pentanol, 1-Hexanol, 2-Propanol, tert-Butanol, 3-Pentanol, Cyclohexanol, Phenol oder Mischungen daraus, Bevorzugt werden Methanol und Ethanol bei der Alkoxycarbonylierung eingesetzt. Wird Methanol eingesetzt, wird die Umsetzung auch als Methoxycarbonylierung bezeichnet. Bei Einsatz von Ethanol wird die Umsetzung auch als Ethoxycarbonylierung bezeichnet.

Das für die Alkoxycarbonylierung eingesetzte homogene Katalysatorsystem umfasst vorzugsweise zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladium-chlorid $[PdCl_2]$, Palladium(II)-Acetylacetonat $[Pd(acac)_2]$, Palladium(II)-Acetat $[Pd(OAc)_2]$, Dichloro-(1,5-cyclooctadien) palladium(II) $[Pd(cod)_2Cl_2]$, Bis(dibenzylideneaceton)palladium(0) $[Pd(dba)_2]$, Tris(dibenzylideneaceton)dipalladium(0) $[Pd_2(dba)_3]$ Bis(acetonitril)-dichloropalladium(II) $[Pd(CH_3CN)_2Cl_2]$, Palladium(cinnamyl)-dichlorid $[Pd(cinnamyl)Cl_2]$. Vorzugsweise kommen die Verbindungen $[Pd(acac)_2]$ oder $[Pd(OAc)_2]$ zum Einsatz. Die Metallkonzentration von Palladium bei der Alkoxycarbonylierung beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann bei der Alkoxycarbonylierung 1 : 1 bis 10 : 1, vorzugsweise 2 : 1 bis 6 : 1, besonders bevorzugt 3 : 1 bis 5 : 1 betragen.

Das homogene Katalysatorsystem umfasst bei der Alkoxycarbonylierung weiterhin eine Säure, wobei es sich insbesondere um eine Brönsted- oder eine Lewis-Säure handeln kann. Als Lewis-Säure können insbesondere Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure. Carbonsäure sind hingegen weniger bis gar nicht geeignet.

Die homogen katalysierte Alkoxycarbonylierung wird vorzugsweise bei einer Temperatur von 25 bis 140 °C, weiterhin bevorzugt bei einer Temperatur von 60 bis 120 °C und besonders bevorzugt bei einer Temperatur von 70 bis 110 °C durchgeführt. Der Druck kann zwischen 5 und 60 bar, vorzugsweise zwischen 10 und 40 bar, besonders bevorzugt zwischen 15 und 30 bar betragen.

Durch die homogen katalysierte Alkoxycarbonylierung wird ein Produktgemisch erhalten, die zumindest den durch die Umsetzung gebildeten Ester, das homogene Katalysatorsystem, Leichtsieder, beispielsweise leichtsiedende Nebenprodukte wie Ether, Hochsieder, nicht umgesetzte Alkohole und ggf. nicht umgesetzte Kohlenwasserstoffe umfasst. Das Produktgemisch kann deshalb einer nachfolgenden Katalysatorabtrennung unterworfen werden. Dies kann beispielsweise mit einer Membrantrennung erfolgen, wodurch das homogene Katalysatorsystem und nicht umgesetzter Kohlenwasserstoff und/oder nicht umgesetzter Alkohol im Retentat angereichert, während der gebildete Ester im Permeat angereichert wird. Das Retentat, welches das angereicherte homogene Katalysatorsystem umfasst, kann in die Reaktionszone zurückgeführt werden.

Die weitere Aufarbeitung des Permeats, insbesondere die Abtrennung der Ester als Zielprodukte, kann nach bekannten Verfahren erfolgen und ist dem Fachmann grundsätzlich geläufig. Eine Möglichkeit sind thermische Trennverfahren wie die Destillation.

Die bei der Destillation in Schritt d) anfallende Leichtsiederphase kann in einer bevorzugten Ausführungsform der vorliegenden Erfindung einer Hydrierung unterworfen werden. Dabei werden die in der Leichtsiederphase vorhandenen Olefine zu den entsprechenden Alkanen hydriert. Die Hydrierung erfolgt in einer Hydriereinheit, die aus einem oder mehreren Reaktoren bestehen kann. Die Reaktoren können im geraden Durchgang oder in Kreislauffahrweise betrieben werden. In einer bevorzugten Ausführungsform der vorliegenden Erfindung umfasst die Hydriereinheit mindestens zwei Reaktoren. Vorzugsweise wird dabei der erste Reaktor im Kreislauf betrieben und der zweite Reaktor im geraden Durchgang. Der erste und der zweite Reaktor können hierbei über einen Überlauf miteinander verbunden sein.

Das hat den Vorteil, dass zwischen dem ersten und dem zweiten Reaktor keine Pumpe eingesetzt werden muss. Bei der Hydrierung wird Wasserstoff vorzugsweise im stöchiometrischen Überschuss eingesetzt, besonders bevorzugt in einem stöchiometrischen Überschuss von 5 bis 30 %.

Die Hydrierung der Leichtsiederphase kann an geeigneten und bekannten Trägerkatalysatoren durchgeführt werden. Geeignete Trägerkatalysatoren umfassen zumindest ein Übergangsmetall aus der Gruppe bestehend aus Palladium, Platin, Rhodium. Ruthenium, Nickel oder Mischungen davon und ein Trägermaterial aus der Gruppe, bestehend aus Aluminiumoxid, Siliciumdioxid, Titandioxid, Magnesiumoxid oder Mischungen davon. In einer bevorzugten Ausführungsform der vorliegenden Erfindung wird bei der Hydrierung im optionalen Schritt ein Trägerkatalysator eingesetzt, der als Übergangsmetall Palladium oder Nickel enthält.

Die Hydrierung wird vorzugsweise bei einer Temperatur von 100 bis 180 °C, besonders bevorzugt bei einer Temperatur von 135 bis 160 °C durchgeführt. Der Druck beträgt bei der Hydrierung vorzugsweise 5 bis 40 barü, besonders bevorzugt 10 bis 30 barü. Der Druck wird hierbei insbesondere durch die Gasphase, also den Wasserstoff erzeugt. Die Hydrierung wird vorzugsweise in der Flüssigphase durchgeführt. Nach der Hydrierung kann eine dem Fachmann bekannte Phasentrennung erfolgen, um die Gasphase, die nicht umgesetzten Wasserstoff und ggf. auch geringe Mengen Kohlenwasserstoffe umfasst, von der flüssigen Phase abzutrennen.

Beispiele

Hydroformylierung von Tributen 49 kg Tributen (Feed) wurden in einer Destillationskolonne (80 l) mit mehreren Packungsbetten (Montz A3-1000) bei einer Temperatur von 70 °C (Kopf) bzw. 110 °C (Sumpf) und einem Druck von 90 mbar (Kopf) bzw. 120 mbar (Sumpf) destilliert. Von den eingesetzten Tributen wurden 20 bis 25% als Destillat (= Leichtsieder) abgetrennt. Die restlichen 75 bis 80% verbleiben im Sumpf und sind daher Schwersieder.

Die drei unterschiedlichen Tributenströme Feed, Destillat und Sumpf wurden auf ihre Zusammensetzung untersucht. Da die Identifizierung der einzelnen Isomere von Tributen aufgrund der hohen Anzahl an Isomeren schwierig ist, wurden die Tributenströme auf den Anteil verschiedener Fraktionen mittels Gaschromatographie (GC-Kapillarsäule, Petrocol, DH 150) untersucht. Zur Vereinfachung wurden deshalb anhand ihrer Retentionszeiten identifiziert (s. auch Abbildung 1):

- Hochverzweigte Isomere mit Retentionszeiten von 70 bis 109 min,
- Mittelverzweigte Isomere mit Retentionszeiten von 109 bis 134 min, und
- Wenigverzweigte Isomere mit Retentionszeiten von 134 bis 180 min

Abbildung 1: Übersicht über die GC-Fraktionen der Tributenströme

Eine Übersicht über den Anteil der jeweiligen o. g. Fraktionen in den Tributenströme ist in Tabelle 1 zu sehen.

Tabelle 1: Anteil der Fraktionen in den jeweiligen Tributenströmen (Massen %)

|  | Feed (vor Destillation) | Destillat (Leichtsieder) | Sumpf (Schwersieder) |
|---|---|---|---|
| Hochverzweigte Isomere | 23,5 % | 93,5% | 0 % |
| Mittverzweigte Isomere | 61 % | 6,5 % | 79 % |
| Wenigverzweigte Isomere | 15,5 % | 0 % | 21 % |

Aus der Analyse von Feed, Destillat und Sumpf zeigt sich, dass durch die Destillation vor allem hochverzweigte Isomere des Tributens aus dem Feed abgetrennt werden und im Destillat landen. Der Anteil an wenigverzweigten Isomeren ist im Sumpf deutlich höher als im Feed oder im Destillat.

Mit den drei Tributenströmen Feed, Destillat und Sumpf wurden in 100 ml Autoklaven jeweils eine Hydroformylierung durchgeführt. Dabei wurde Rhodium (40 ppm Rh) mit einem Liganden (Alkanox 240) im 5-fachem molaren Überschuss ((Verhältnis Gesamtphosphor zu Rhodium) als Katalysator eingesetzt. Die Temperatur betrug 130 bis 150 °C. Die Hydroformylierung wurden weiterhin jeweils bei 235 bis 250 bar Synthesegasdruck (CO/$H_2$-Verhältnis = 1:1 (Vol.-%)) durchgeführt. Als Lösemittel wurden jeweils 40 bis 46 g Toluol verwendet. 10 Minuten, 60 Minuten und 180 Minuten nach Reaktionsstart wurden jeweils Proben genommen und auf den Reaktionsumsatz untersucht (Umsatz= Stoffmenge zum Zeitpunkt t/ Stoffmenge (Beginn der Reaktion)). Die Ergebnisse der Hydroformylierung sind in Tabelle 2 gezeigt.

Tabelle 2: Reaktionsumsatz bei der Hydroformylierung

|  | Umsatz / % | | |
|---|---|---|---|
|  | Nach 10 Minuten | Nach 60 Minuten | Nach 180 Minuten |
| Feed (vor Destillation) | 38,8 | 80,4 | 90,9 |
| Destillat (Leichtsieder) | 5,9 | 32.51 | 57,8 |
| Sumpf (Schwersieder) | 43,3 | 85.12 | 94,2 |

Aus der Tabelle 2 ist zu entnehmen, dass eine deutliche Beschleunigung der Reaktion durch eine vorherige Destillation erreicht werden kann. Verglichen mit dem Feed ist der Umsatz bei Einsatz des Sumpfstroms jeweils um etwa 5% höher.

Hydroformylierung von Dibuten

A) Umsetzung in einer Stufe

21,5 t/h Dibuten (Feed) wurden in einer Destillationskolonne mit 42 Böden bei einer Kopftemperatur von 58 °C und einer Sumpftemperatur von 62 °C und entsprechenden Kopfdruck von 0,1 bar und einem Sumpfdruck von 0,15 bar destilliert. Von dem eingesetzten Dibuten wurden etwa 20% als Destillat (= Leichtsieder) abgetrennt (4,44 t/h). Die restlichen 80% wurden im Sumpf abgenommen und sind daher Schwersieder.

Die Dibutenströme Feed und Sumpf wurden mittels GC (Gaschromatographie) auf ihre Zusammensetzung untersucht. Eine Übersicht ist in Tabelle 3 gezeigt:

Tabelle 3: Anteil verschiedener Gruppen von Isomeren im jeweiligen Dibutenstrom

|  | Feed (vor Destillation) | Sumpf (Schwersieder) |
|---|---|---|
| Dimethylhexene | 19 % | 8 % |
| Methylheptene | 65 % | 72 % |
| Octene | 16 % | 20 % |

Die Linearität von Dibuten wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach der folgenden allgemeinen Formel, wobei sich der Anteil der einzelnen Dimerfraktionen auf die Gesamtdimer-Fraktion bezieht:

$$ISO - Index = \frac{(\text{einfach verzweigte Dimere (Gew.-\%)} + 2 \times \text{zweifach verzweigte Dimere (Gew.-\%)})}{100}$$

Demnach besitzt ein Dimerengemisch mit einem ISO-Index von 1,0 im Mittel genau eine Methylverzweigung pro Dimerenmolekül.

Der ISO-Index des Feeds beträgt 1,03 und der ISO-Index des Sumpfes beträgt 0,88. Es zeigt sich, dass der Sumpf einen höheren Anteil linearer Isomere enthält.

Mit dem Feed und dem Sumpf wurden in 100 ml Autoklaven jeweils eine Hydroformylierung durchgeführt. Dabei wurde Rhodium (20 ppm Rh) mit einen Liganden (Tris-(2,4-di-tert.-butylphenyl)-phosphit (TDTBPP)) im 5-fachem molaren Überschuss ((Verhältnis Gesamtphosphor zu Rhodium) als Katalysator eingesetzt. Die Temperatur betrug etwa 140 °C. Die Hydroformylierung wurden weiterhin jeweils bei etwa 235 bar Synthesegasdruck ($CO/H_2$-Verhältnis = 1:1 (Vol.-%)) durchgeführt. Als Lösemittel wurden jeweils 150 ml Toluol verwendet. 10 Minuten, 30 Minuten, 60 Minuten und 180 Minuten nach Reaktionsstart wurden jeweils Proben genommen und auf den Reaktionsumsatz untersucht (Umsatz= Stoffmenge zum Zeitpunkt t/ Stoffmenge (Beginn der Reaktion)). Die Ergebnisse der Hydroformylierung sind in Tabelle 4 gezeigt.

Tabelle 4: Reaktionsumsatz bei der Hydroformylierung der Dibutenströme

| | Umsatz / % | | | |
|---|---|---|---|---|
| | Nach 10 Minuten | Nach 30 Minuten | Nach 60 Minuten | Nach 180 Minuten |
| Feed (vor Destillation) | 51 | 79.9 | 90 | 92.5 |
| Sumpf (Schwersieder) | 71 | 89.4 | 94 | 95.1 |

Aus der Tabelle 4 ist zu entnehmen, dass eine deutliche Beschleunigung der Reaktion durch eine vorherige Destillation erreicht werden kann. Verglichen mit dem Feed ist der Umsatz bei Einsatz des Sumpfstroms jeweils um bis zu 20% höher.

B) Umsetzung in einer zweiten Stufe

Die Hydroformylierung wurde wie unter A) beschrieben durchgeführt. Der Unterschied war, dass ein zweiter Reaktor vorhanden war, in dem die Hydroformylierung durchgeführt wurde.

Von dem eingesetzten Dibuten wurden etwa 24% als Destillat (= Leichtsieder) abgetrennt. Die restlichen 76% wurden im Sumpf abgenommen und sind daher Schwersieder. Die Dibutenströme Feed, Destillat und Sumpf wurden mittels GC (Gaschromatographie) auf ihre Zusammensetzung untersucht. Eine Übersicht ist in Tabelle 5 gezeigt:

Tabelle 5: Anteil verschiedener Gruppen von Isomeren im jeweiligen Dibutenstrom

| | Feed (vor Destillation) | Destillat (Leichtsieder) | Sumpf (Schwersieder) |
|---|---|---|---|
| Dimethylhexene | 37 % | 51 % | 30 % |
| Methylheptene | 58 % | 48 % | 64 % |
| Octene | 5 % | 1 % | 6 % |

Mit allen drei Strömen wurden Hydroformylierungen durchgeführt. Die Temperatur betrug 180 °C, der Synthesegasdruck 265 bar. Die Ergebnisse der Hydroformylierung sind in Tabelle 6 gezeigt.

Tabelle 6: Reaktionsumsatz bei der Hydroformylierung in der zweiten Stufe

| | Umsatz / % |
|---|---|
| | Nach 180 Minuten |
| Feed (vor Destillation) | 72,9 |
| Destillat (Leichtsieder) | 70,7 |
| Sumpf (Schwersieder) | 77,6 |

Aus der Tabelle 6 ist zu entnehmen, dass eine deutliche Beschleunigung der Reaktion durch eine vorherige Destillation erreicht werden kann. Verglichen mit dem Feed ist der Umsatz bei Einsatz des Sumpfstroms jeweils um bis zu etwa 5 % höher.

**Patentansprüche**

1. Verfahren zur Umsetzung von Olefinen mit 4 bis 20 Kohlenstoffatomen, welches die folgenden Schritte umfasst:

   a) Bereitstellen eines Feedstroms, der zumindest lineare Olefine und verzweigte Olefine mit jeweils gleicher Anzahl an Kohlenstoffatomen umfasst;
   b) Zuführen des Feedstroms aus Schritt a) zu einer Reaktionseinheit, die einen oder mehrere Reaktoren umfasst, und Durchführen einer Umsetzung in der Reaktionseinheit unter Erhalt eines Produktgemisches, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6-Alkohol ist,
   c) Abtrennen eines Reststroms aus dem Produktgemisch aus Schritt b), wobei der Reststrom zumindest nicht umgesetzte Olefine umfasst, aus dem Reaktionsgemisch;
   d) Destillation des Reststroms aus Schritt c) in mindestens einer Destillationskolonne unter Erhalt zumindest einer Leichtsiederphase und einer Schwersiederphase, wobei 1 Gew.-% bis weniger als 50 Gew.-% des destillierten Feedstroms als Leichtsiederphase anfällt und wobei der Anteil an linearen Olefinen in der Leichtsiederphase geringer ist als der Anteil der linearen Olefine in der Schwersiederphase; und
   e) Zuführen der Schwersiederphase aus Schritt d) zu einer Reaktionseinheit, die einen oder mehrere Reaktoren umfasst, und Durchführen einer Umsetzung in der Reaktionseinheit, wobei die Umsetzung entweder eine heterogen katalysierte Hydroformylierung in Anwesenheit von Synthesegas, eine homogen katalysierte Hydroformylierung in Anwesenheit von Synthesegas oder eine homogen katalysierte Alkoxycarbonylierung in Anwesenheit von Kohlenmonoxid und einem C1- bis C6-Alkohol ist.

2. Verfahren nach Anspruch 1, wobei bei dem Verfahren Olefine mit 7 bis 16 Kohlenstoffatomen, vorzugsweise 7 bis 12 Kohlenstoffatomen umgesetzt werden.

3. Verfahren nach Anspruch 1 oder 2, wobei das homogene Katalysatorsystem bei der Hydroformylierung Co oder Rh und vorzugsweise einem phosphorhaltigen Liganden umfasst.

4. Verfahren nach Anspruch 1 oder 2, wobei das homogene Katalysatorsystem bei der Alkoxycarbonylierung ein Metall aus der Gruppe 8 bis 10 des Periodensystems der Elemente (PSE) oder eine Verbindung davon, einen phosphorhaltigen Liganden und eine Säure als Co-Katalysator umfasst.

5. Verfahren nach einem der vorhergehenden Ansprüche, wobei die in Schritt d) anfallende Leichtsiederphase einer Hydrierung unterworfen wird.

6. Verfahren nach Anspruch 5, wobei die Hydrierung bei einer Temperatur von 100 bis 180 °C durchgeführt wird.

7. Verfahren nach Anspruch 5 oder 6, wobei bei der Hydrierung Wasserstoff im stöchiometrischen Überschuss, vorzugsweise in einem stöchiometrischen Überschuss von 5 bis 30 % eingesetzt wird.

8. Verfahren nach einem der Ansprüche 5 bis 7, wobei bei der Hydrierung ein Trägerkatalysator, umfassend zumindest ein Übergangsmetall aus der Gruppe bestehend aus Palladium, Platin, Rhodium. Ruthenium, Nickel oder Mischungen davon und ein Trägermaterial aus der Gruppe, bestehend aus Aluminiumoxid, Siliciumdioxid, Titandioxid, Magnesiumoxid oder Mischungen davon, eingesetzt wird.

9. Verfahren nach einem der vorhergehenden Ansprüche, wobei die Destillation in Schritt d) bei einer Kopftemperatur im Bereich von 40 bis 100 °C, bevorzugt im Bereich von 55 bis 80 °C durchgeführt wird.

10. Verfahren nach einem der vorhergehenden Ansprüche, wobei der Druck am Kopf bei der Destillation in Schritt d) 50 bis 400 mbar, bevorzugt 80 bis 350 mbar beträgt.

11. Verfahren nach einem der vorhergehenden Ansprüche, wobei die mindestens eine Destillationskolonne in Schritt d) 10 bis 100 Böden, bevorzugt 15 bis 80 Böden umfasst.

**12.** Verfahren nach einem der vorhergehenden Ansprüche, wobei in Schritt d) 5 Gew.-% bis 40 Gew.-%, vorzugsweise 10 Gew.-% bis 30 Gew.-% des destillierten Feedstroms als Leichtsiederphase anfällt.

**13.** Verfahren nach einem der vorhergehenden Ansprüche, wobei die Abtrennung des Reststroms in Schritt c) mittels thermischer Trennung erfolgt, wobei die thermische Trennung vorzugsweise eine Destillation, eine Dünnschichtverdampfung, eine Fallfilmverdampfung oder eine Kombination von zwei oder mehr davon ist.

**14.** Verfahren nach einem der vorhergehenden Ansprüche, wobei der Umsatz bei der Umsetzung in Schritt b) maximal 95%, vorzugsweise maximal 92%, weiterhin bevorzugt maximal 90%, weiterhin bevorzugt maximal 88%, besonders bevorzugt maximal 85% beträgt.

Europäisches
Patentamt

European
Patent Office

Office européen
des brevets

**EUROPÄISCHER RECHERCHENBERICHT**

Nummer der Anmeldung

EP 24 21 8156

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 1 172 349 A2 (OXENO OLEFINCHEMIE GMBH [DE]) 16. Januar 2002 (2002-01-16) * Absätze [0001], [0004] - [0006] * * Absatz [0013] * * Abbildung 1; Beispiel 3 * ----- | 1-14 | INV. C07C7/04 C07C11/02 C07C45/50 C07C47/02 C07C67/38 C07C69/24 |
| A | EP 1 029 839 A1 (OXENO OLEFINCHEMIE GMBH [DE]) 23. August 2000 (2000-08-23) * Ansprüche; Beispiele * ----- | 1-14 | |
| A | ERNST BILLIG ET AL: "Oxo Process", 2000, KIRK-OTHMER ENCYCLOPEDIA OF CHEMICAL TECHNOLOGY, PAGE(S) 1 - 17, XP007918022, DOI: 10.1002/0471238961.15241502091212.a01, * das ganze Dokument * * Seite 2, Absatz 4 * ----- | 1-14 | |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| Den Haag | 10. April 2025 | Kardinal, Siegmar |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 24 21 8156

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2025

| Im Recherchenbericht angeführtes Patentdokument | Datum der Veröffentlichung | Mitglied(er) der Patentfamilie | Datum der Veröffentlichung |
|---|---|---|---|
| EP 1172349 A2 | 16-01-2002 | AR 029715 A1 | 10-07-2003 |
| | | AT E293588 T1 | 15-05-2005 |
| | | BR 0102718 A | 26-02-2002 |
| | | CA 2353061 A1 | 14-01-2002 |
| | | CN 1333201 A | 30-01-2002 |
| | | CZ 20012521 A3 | 13-03-2002 |
| | | DE 10034360 A1 | 24-01-2002 |
| | | EP 1172349 A2 | 16-01-2002 |
| | | ES 2238363 T3 | 01-09-2005 |
| | | JP 5322073 B2 | 23-10-2013 |
| | | JP 2002053501 A | 19-02-2002 |
| | | KR 20020008020 A | 29-01-2002 |
| | | MX PA01007093 A | 19-10-2005 |
| | | MY 121183 A | 30-12-2005 |
| | | PL 348628 A1 | 28-01-2002 |
| | | RO 120842 B1 | 30-08-2006 |
| | | RU 2296739 C2 | 10-04-2007 |
| | | SG 95649 A1 | 23-04-2003 |
| | | TW 574197 B | 01-02-2004 |
| | | US 2002028974 A1 | 07-03-2002 |
| | | ZA 200105781 B | 22-01-2002 |
| EP 1029839 A1 | 23-08-2000 | AR 022612 A1 | 04-09-2002 |
| | | BR 0000487 A | 12-09-2000 |
| | | CA 2298871 A1 | 17-08-2000 |
| | | CN 1266835 A | 20-09-2000 |
| | | DE 19906518 A1 | 31-08-2000 |
| | | EP 1029839 A1 | 23-08-2000 |
| | | ID 24790 A | 16-08-2000 |
| | | JP 2000239196 A | 05-09-2000 |
| | | KR 20000058062 A | 25-09-2000 |
| | | PL 338467 A1 | 28-08-2000 |
| | | SG 89317 A1 | 18-06-2002 |
| | | TW 491827 B | 21-06-2002 |
| | | US 6433242 B1 | 13-08-2002 |
| | | ZA 200000733 B | 08-09-2000 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3632885 A1
- EP 3750620 A1
- WO 2015028284 A1
- EP 3121184 A2


**In der Beschreibung aufgeführte Nicht-Patentliteratur**

- **P. W. N. M VAN LEEUWEN**. Rhodium Catalyzed Hydroformylation. 2002
- **A. BÖRNER** ; **R. FRANKE**. Hydrofomylation - Fundamentals, Proceses and Applications in Organic Synthesis. 2016